# EUROPEAN PATENT SPECIFICATION

(11) **EP 0 532 805 B1**
(45) Date of publication and mention of the grant of the patent: **08.10.1997**
(21) Application number: 91308574.2
(22) Date of filing: 20.09.1991
(51) Int. Cl.: A61F 13/58

(54) **Article having target part for adhering and method for producing it**
Artikel mit Empfangsteil für Klebeverschluss sowie Verfahren zu dessen Herstellung
Article pourvu d'une partie réceptrice pour l'adhésion et procédé de fabrication

(43) Date of publication of application: 24.03.1993
(73) Proprietor: NITTA GELATIN INC., Osaka-shi Osaka 541 (JP)
(72) Inventor: Matsumoto, Mutsuo, Yamatotakada-shi, Nara 635 (JP); Murotani, Yasuyoshi, Fujiidera-shi, Osaka 583 (JP); Takemiya, Hidenori, Nara-shi, Nara 631 (JP)
(74) Representative: Pacitti, Pierpaolo A.M.E.

(56) References cited:
- EP-A- 0 440 163
- FR-A- 2 617 682
- GB-A- 2 054 350
- GB-A- 2 135 568

## Description

### BACKGROUND OF THE INVENTION

The present invention relates to an article having first and second portions, the first portion having a target part and the second portion having a tab to enable the tab to be releasably attached to the target part, and to a method for producing it. Examples of such an article include disposable diapers, etc.

A representative example of an article having a target part for adhering is a disposable diaper. The diaper, when worn, has a fastening system to enable the diaper to be fixed to a body. A fastening system of this type consists of a tab having an adhering face and a target part to which the tab adheres so as to form an impermanent adhesion which can be terminated and re-established repeatedly. Examples of such fastening systems may be found in UK Patent Application No 2054350, European Patent Application No 0440163, UK Patent Application No 2135568 and French Patent Application No 2617682. The target part is usually arranged on an outer face of the front of the diaper. Thus, the tab arranged at both sides of the rear of the diaper is able to adhere with the target part and to peel off from it. That is, the diaper is put on the part of the body where it is worn and is set temporarily so as to decide a position for wearing, then fixed, and used. When the diaper is taken off, the above described fastening system is released. The target part is needed because the surface of a base material is prepared so as to satisfy such properties as water-nonpermeability, flexibility, and fashion appearance, the surface is not suitable to adhere to the tab repeatedly.

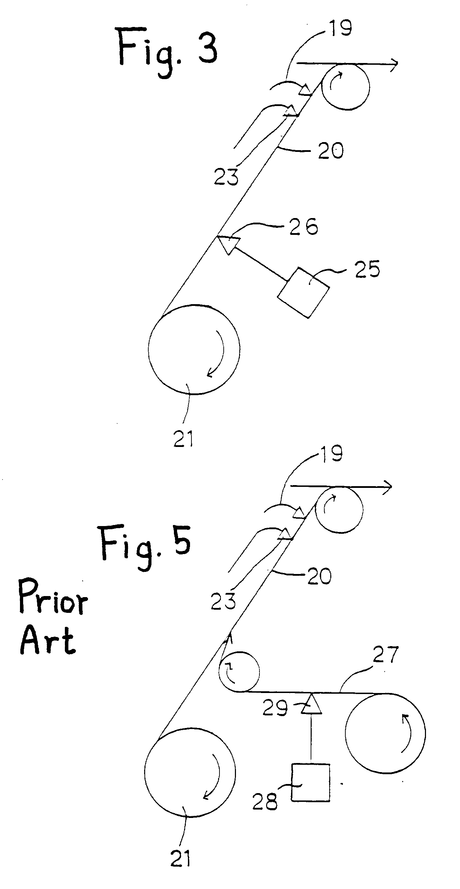

This target part, for example, as shown in Fig. 4, is made by sticking a resin sheet 202 on a base 200 by means of an adhering agent 201. A method for producing it is, for example, constituted as follows. In a previous method for producing a diaper, Fig. 5 typically shows one example of a process for making the target part. As shown in Fig. 5, an oriented polypropylene film 27 is continuously supplied and, on the whole of a rear face of the film 27, a hot melt adhesive 28 is continuously coated with a gun for coating 29. This film is cut in a proper size and stuck together on a surface which becomes the outer face side of the polyethylene film 20 being continuously supplied. Thus, it turns out that the target part is made on the outer face of the polyethylene film 20.

Otherwise, there is the case where an adhering tape, the surface of which has been subjected to mold-releasing treatment ( needless to say, on the back of which a release paper is stuck ), is purchased and the tape is stuck on the outer face of the polyethylene film.

In the forementioned conventional procedure, the material cost for making the target part may be high and the process for making it may be complex and, therefore, the total cost is usually a great deal.

### SUMMARY OF THE INVENTION

Thus, the first object of the present invention is to provide an article which comprises reduction of the total cost of making a target part and having a target part for adhering, and the second object is to provide a method for producing an article of the above type continuously.

According to the present invention there is provided an article having a target part and a tab to attach to the target part to form an impermanent adhesion therewith which can be terminated and re-established repeatedly, the target part having thereon a hot melt resin composition, characterised in that the hot melt resin composition has an open time of 0.5 seconds or less and comprises
i) 20-40% by weight of a base polymer;
ii) 20-40% by weight of a tackifier resin;
iii) 30-50% by weight of Sasol Wax H1;
iv) 0-20% by weight of an oil:
and in that the coating of the hot melt resin composition on the target part has a pattern which defines alternative attachment areas on the target part for attachment of the tab.

According to a second aspect of the present invention there is provided a method for producing an article as hereinbefore defined, characterised in that the method comprises continuously moving the target part and applying the hot melt resin composition directly onto the continuously moving target part.

If, as in the first aspect of the invention, the open time is longer than 0.5 seconds, there may be a problem of adhesive-attaching to a press roll.

Preferably, screen coating is used to coat the hot melt resin composition onto said surface.

Preferably, the pattern of the hot melt resin composition coated on the material surface indicates a position for the location of said tab.

It is preferred that the hot melt resin composition used in the present invention is satisfactory for all the following conditions from 1 to 3 .
① The surface energy of a solidified matter is small. A preferable surface energy is in a range of from about 15 to 25 dyne/cm² in a point of view that facility in adhering with the adhering face of a tab to be firmly fixed is compatible with the facility in peeling-off from it. If the surface energy is bigger out of the range, the mold-releasing character is lost and the tape release may becomes difficult. If smaller, the tape may not adhere.
② The adhering character with a base material is excellent. Even if the adhering with a tab to be firmly fixed as well as the peeling-off from it are repeated many times, it is required that the peeling-off takes place at an interface between the firmly fixed tab and the target part, but not at an interface between the target part and the base material.
③ A blocking character is absent. A preferable blocking character is not to cause that the target part for adhering is not peeled off due to attaching to other matter in a period of time between the target formation and practical use. It is also preferred that the blocking character absence is exhibited, for example, at about 65°C or less.

The hot melt resin composition is prepared so as to be satisfactory for all the above-mentioned conditions by combining, in a suitable proportion, a base polymer, a tackifier resin, wax, oil, and an antioxidant, etc.

As the base resin is used, for example, one kind or two or more of the thermoplastic resin such as a copolymer of ethylene-vinyl acetate ( EVA ), polystyrene-polybutadiene-polystyrene block copolymer ( SBS ), polystyrene-polyisoprene-polystyrene block copolymer ( SIS ), polystyrene-polyethylene/polybutylene-polystyrene block copolymer ( SEBS ), and a polyolefin-based polymer.

As the tackifier resin is used, for example, one kind or two or more of an aliphatic or alicyclic hydrocarbon resin, rosin and its derivative, and a terpene-based resin.

As the oil is used, for example, one kind or two or more of paraffin-based oil and naphthene-based oil.

Besides, in a case where a hot melt resin composition is used in a place where people look, coloring into a desired color is possible using an appropriate pigment for coloring.

Among the above components, the base polymer, tackifier resin, wax, and oil are used, for example, in the following proportion. Against 100 parts by weight of a total of these four components, the base polymer is from about 20 to 40 parts by weight, the tackifier resin from about 20 to 40 parts by weight, the wax from about 30 to 50 parts by weight, and the oil from about 0 to 20 parts by weight. Besides, in a case where an antioxidant is used, it is preferred to use in a proportion of 1.0 part by weight or less against the forementioned 100 parts, and in a case where a pigment for coloring is used, it is preferred to use in a proportion of 1.0 part by weight against that.

Furthermore, according to the present invention, since the target part is made by a hot melt resin composition, various properties can be afforded by properly designing the hot melt resin composition. For example, the properties comprises adjustment of flexibility, reinforcement of a base material, control of a peel strength. The property-affording can be carried out, for example, by controlling an adding amount of a rubber-based polymer and by an use of a resin having a high softening point or of wax.

A hot melt resin composition prepared with the above-described components is directly coated on a base material surface. This coating is carried out by using a common hot melt coating gun. An add-on level is decided depending upon the size of a forming target part. The coating thickness may be similar to the case of a common hot melt adhesive, for example, it is in an order of from about 50 to 200 µm. Also, since the target part is made by coating the hot melt resin composition, it can be easily made even for a continuously moving base material. Furthermore, since there is no process of lowering a line speed such as cutting of an adhesive tape, it is possible to quicken the line speed. Of course, it is unnecessary to carry out formation of the target part with continuous moving.

In a case where an article having a target part for adhering is used repeatedly, it is troublesome to determine a position for sticking a tab on the target every time the article is set. If a mark to indicate the position for sticking the tab is used, after determining the position by the mark and then, using this mark, the tab can be easily stuck at the defined position.

In a case where a hot melt resin composition to make a target part is coated on the whole face of a part which becomes a substrate of the target part on a base material surface, if a mark showing a position to stick a tab on the base material surface is beforehand printed, it is convenient for use because the mark printed on the base material surface is seen through a film of the composition.

If such a mark is not printed, the mark can be drawn by a hot melt resin composition when this composition is coated. In a case like this, screen coating is very effective. According to the screen coating, a screen having many small holes is used to draw a desired pattern and a hot melt resin composition is pushed out through the holes to perform coating with resulting small dots and, thus, a part of the coated pattern is a mark showing a position for sticking a tab. In this case, the coated pattern is an assembly of dots and it is possible to make various designs. Also, by changing the pattern shape and/or the dot size variously, even if adhesive strength of a tab adhesive is various, the peel strength with a stuck tab can be adjusted so as to be in a proper strength without any change in the components and combination of a hot melt resin composition.

As the base material to make the target part are used a polyethylene film and OPP ( an oriented polypropylene ) film in a case where a disposable paper is produced.

Articles having the target part for adhering and produced by the present invention are, for example a disposable diaper and a drape for medical use, but the article is not limited with those described here.

Besides, the " tab " means, in an article having a target part, that part arranged on the article with an adhering face to adhere with the target part repeatedly. For example, when a type of an adhesive tape adheres, on a part of it, with a body to be stuck and its other part is free without being stuck, this free part is called as a tab. Furthermore, when the adhesive character needs not to be displayed, a release paper may be stuck to maintain the adhesive character of the tab. In this case, the release paper is peeled off and the tab adheres with the target part.

By making the target part by a hot melt resin composition, it is unnecessary to use a resin sheet which converts into the target part and, therefore, the material cost decreases.

When the hot melt resin composition is directly coated on a base material, a process to supply a resin sheet converting into the target part becomes unnecessary and the process is simplified. In addition, conventional continuous production line can be used.

The article having a target part for adhering and relating to the present invention, as mentioned above, is able to intend a cost decrease in a point of materials and also, in a point of the simplified producing device.

The article having a target part for adhering and relating to the present invention, as mentioned above, is able to intend a cost decrease in a point of materials and also, in a point of the simplified producing device.

Hereinafter, the present invention is in detail explained referring to figures which show examples, but the present invention is not limited with the examples and figures.

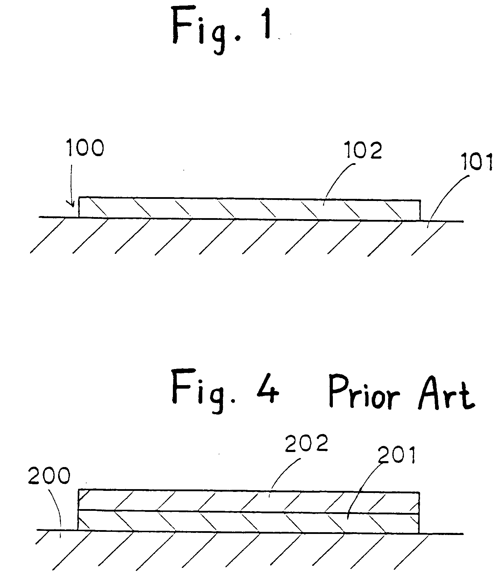

Fig. 1 is a cross-sectional view showing a part in an example of an article having a target part for adhering and relating to the present invention. As seen in Fig. 1, this article 100 has a target part 102 made by a hot melt resin composition on a surface of the base material 101 and, when compared with a conventional target part shown in Fig. 4, the material is in a less amount by the portion of the resin sheet 202. Such an article 100 may be produced by a continuous production method or a noncontinuous production method.

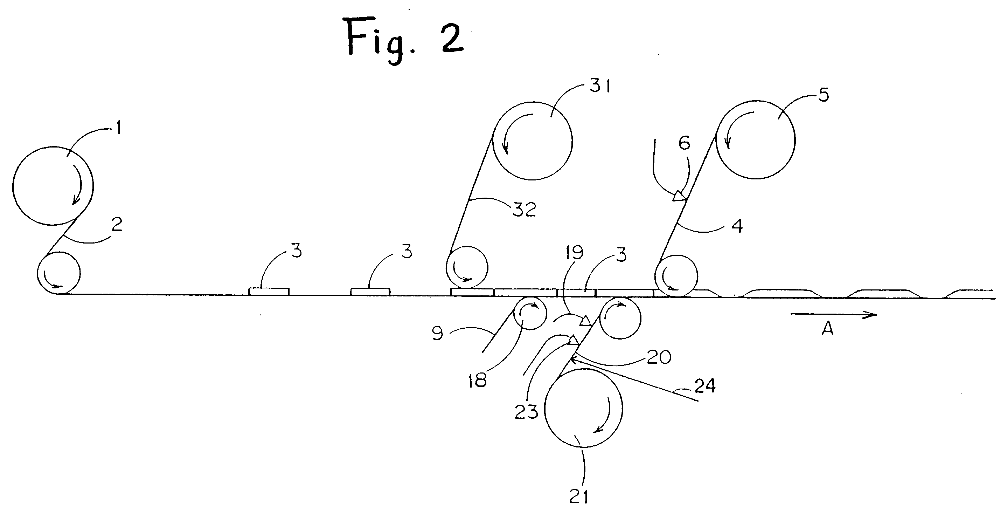

Fig. 2 typically shows an example of the present invention in a case where the method for producing an article having a target part for adhering is a method for producing a disposable diaper. As seen in this figure, a tissue paper 2 is continuously supplied from the roll 1 and a tissue paper 32 is continuously supplied from the roll 31, and a water-absorbent material 3 is put between these tissue papers 2 and 32. A polyethylene film 20 ( which becomes a back sheet ) is continuously supplied from the roll 21 and, as seen in Fig. 3, a hot melt resin composition 25 is in a necessary amount coated on the polyethylene film 20 by the gun for coating 26 ( shown by 24 in Fig. 2 ), and thereby, a target part ( not shown in the figure ) is made on an outer surface of the polyethylene film 20. This hot melt resin composition 25 is such as the above-mentioned. The polyethylene film 20 having the already formed target is continuously supplied and, on the rear side of this film 20, hot melt adhesives supplied from the guns for coating 19 and 23 are continuously coated. Thus, the film 20 is stuck on the tissue paper 2 by the adhesive so that it greatly overhangs on this tissue paper 2. Then, an elastic band 9 is stuck together by the adhesive so as to form a stretching and shrinking part on both sides of the water-absorbent material 3. On the other hand, a non-woven fabric 4 is continuously supplied from the roll 5, the adhesive is continuously supplied on the non-woven fabric 4 from the gun for coating 6, the non-woven fabric 4 is stuck on another tissue paper 32 by the adhesive so that it greatly overhangs on this tissue paper 32. Thus, there is obtained a matter that a sandwich-like body such as a combination of tissue paper 2-water-absorbent material 3-tissue paper 32 is put between the polyethylene film 20 and the non-woven fabric 4. This matter is cut out between each piece of the sandwich-like body, and both the sides of each sandwich are cut so as to gouge out in a curved shape in order to obtain a disposable diaper. Although formation of a tab having an adhering face has not been mentioned, it is carried out in an usual way.

The hot melt adhesive is applied from the guns for coating 6, 19, and 23 like drawing many lines.

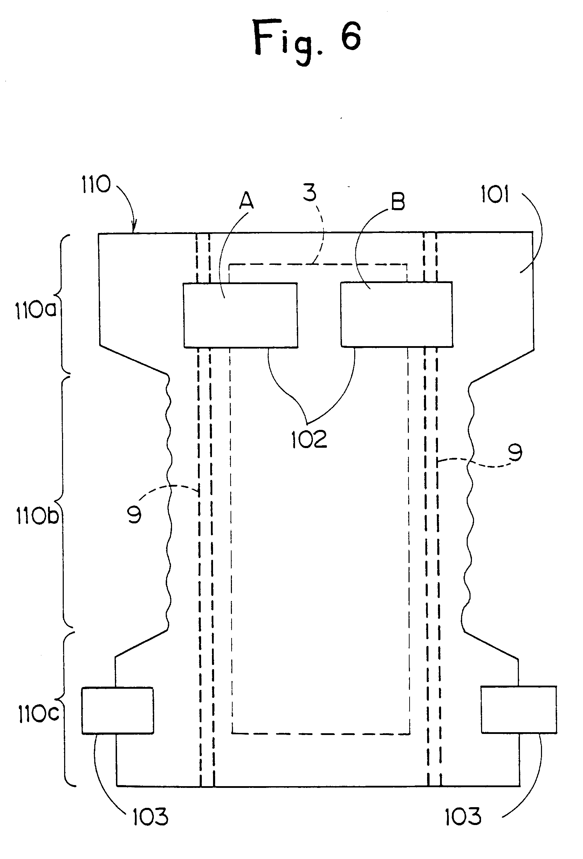

Fig. 6 shows a plan view of an example in a case where the article in the present invention is a disposable diaper.

As seen in Fig. 6, this diaper 110 has a I-character form and is made up, in sequence from the above, of the lower abdomen part 110a having two wings stretched to the left and right, the crotch part 110b constricted at the central part, and the hip part 110c having two wings stretched to the left and right in a lower side of the crotch 110b. In an area of from the lower abdomen 110a to the hip 110c, a thing made by holding an absorbent material 3 between two tissue papers is received, so that urine etc. is absorbed and held. On both left and right sides of the absorbent material 3 of the crotch 110b, an elastic band 9 is arranged in upper and lower directions, so that a shrinking force works when the band 9 is stretched toward a length direction and thus, when the diaper 110 is set, it is in firm contact around the groin of a body. The tab 103 is set at the pointed ends of two wings of the hip 110c. On a surface of a base material ( for example, a polyethylene film ) of the lower abdomen part 110a, the target part for adhering 102 is made of a hot melt resin composition as described above.

If a mark is beforehand printed to indicate a tab-sticking position on a surface of the base material 101 on a lower side of the target part for adhering 102, the mark printed on the base material 101 is seen through a film of the hot melt resin composition.

On the other hand, when the target part 102 is made by coating the hot melt resin composition, the mark may be drawn with the hot melt resin composition.

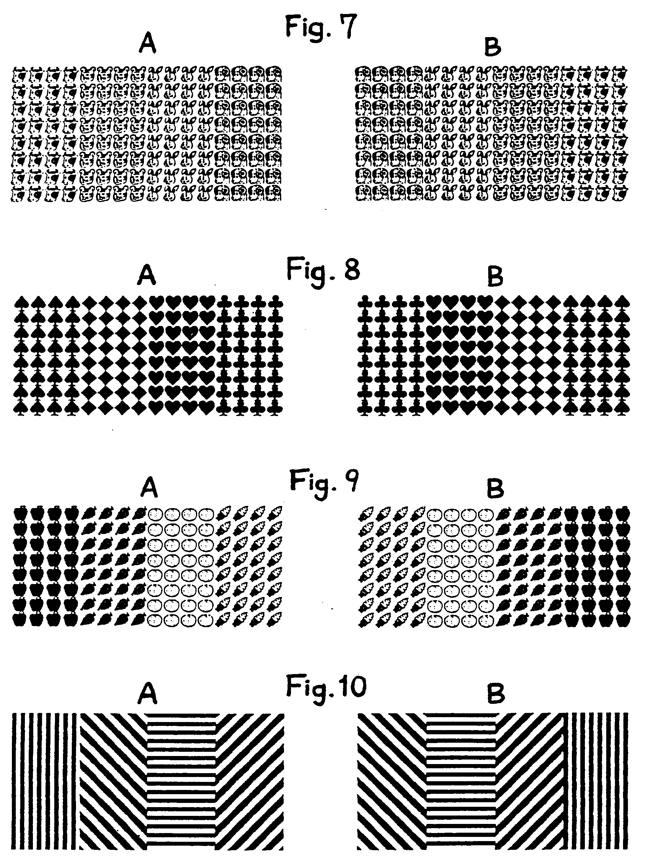

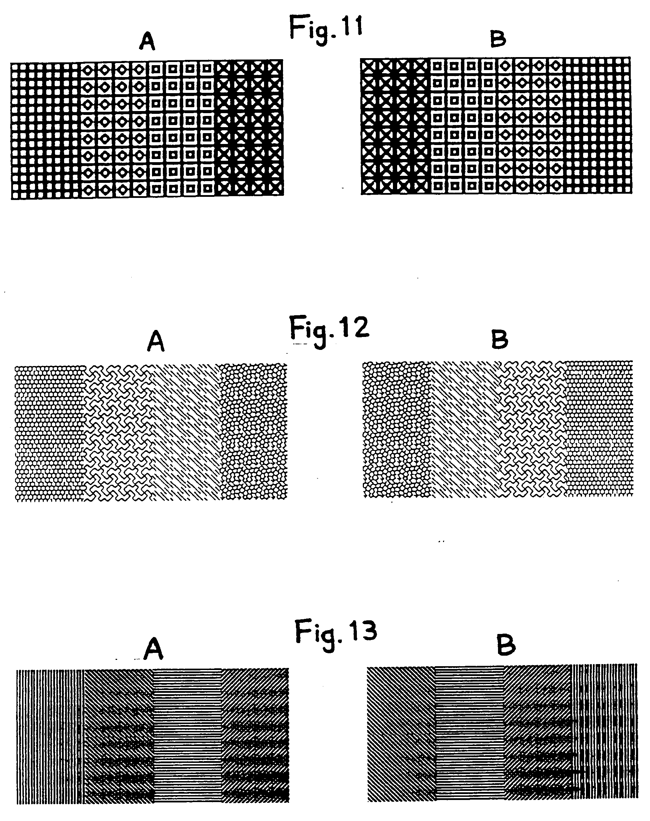

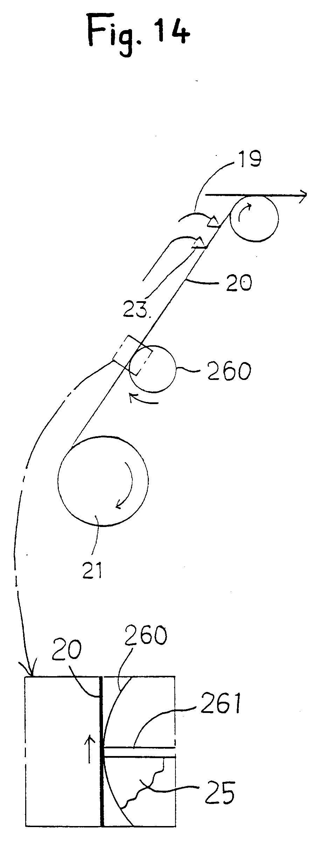

Figs. 7 ∼ 13 show pattern examples of the left and right target parts 102 which are made so as to have such a mark. When the disposable diaper 110 is set, each of A and B in Figs. 7 ∼ 13 is arranged so as to come, respectively, to the right lower abdomen and left lower abdomen. In Figs. 7 ∼ 13, as the tab-sticking position is allowed to come near the center, the fastening becomes more tight, and as it is allowed to come near the outside, the fastening becomes looser. In a case where the same disposable diaper is repeatedly applied for the same person, if a mark in the most suitable position is once remembered, sticking of the tab 103 at a position of the mark is easily set from the next time.

Fig. 14 shows an example in a case where a target part for adhering is made by a screen printing of the hot melt resin composition. Here, instead of the gun for coating 26 in Fig. 3 is used a cylinder type screen 260 which is arranged so as to circulate around the center axis. When the hot melt resin composition 25 for making a target part for adhering is placed in the cylinder type screen 260 and this screen is circulated, the hot melt resin composition 25 is pressed to the inner circumference face by a doctor blade 261 etc., passes through transmission holes to the outside, and then, is coated on a surface of the polyethylene film 20, that is a base material. In this case, the coating pattern of the hot melt resin composition 25 is, for example, as shown in Figs. 7 ∼ 13.

The cylinder type screen 260 is, for example, designed so that one circumference length corresponds to a positive integer time as long as the disposable diaper 110, and the transmission holes are arranged, for example in a pattern shown Figs. 7 ∼ 13, in a part of a circular arc as long as one disposable diaper, wherein the part corresponds to the target part for adhering. Having such arrangements, if a screen printing is carried out by circulating the screen 260 in concert with velocity of a disposable diaper-producing line, a process for making the target part for adhering 102 can be included in the disposable diaper-producing line. Of course, adopting a different method, the target part for adhering may be made by a screen printing.

### BRIEF DESCRIPTION OF THE DRAWING

Fig. 1 is a cross-sectional view showing a part in an example of an article relating to the present invention and having a target part for adhering.

Fig. 2 typically shows an example in a case where the method for producing an article relating to the present invention and having a target part for adhering is a method for producing a disposable diaper.

Fig. 3 typically shows a principal part of the example in Fig. 2.

Fig. 4 is a cross-sectional view showing a previous target part.

Fig. 5 typically shows a process for making a previous target part.

Fig. 6 is a plan view of an example in a case where an article having a target part for adhering in the present invention is a disposable diaper.

Figs. 7 ∼ 13, respectively, show pattern examples of the target part for adhering.

Fig. 14 typically shows another example of the principal part in Fig. 2.

### DESCRIPTION OF THE PREFERRED EMBODIMENTS

Hereinafter, practical examples and comparative examples of the present invention are shown, but this invention is not limited with the undermentioned examples.

### Example 1

With the devices shown in Figs. 2 and 3, a disposable diaper was produced as mentioned above. The hot melt resin composition used for making the target part for adhering had the following composition.
The hot melt resin composition was,

| | |
|---|---|
| base polymer : EVA ( EV-220, made by Mitsui Dupont Polychemical Co., Ltd. ) | 33 parts by weight |
| tackifier resin : C₉ -based hydrogenated petroleum resin ( Arkon M115, made by Arakawa Chemical Industries, Ltd. ) | 22 parts by weight |
| wax : ( Sasol H 1, made by Sasol Chemical Industries, Ltd. ) | 45 parts by weight |
| antioxidant ( age resister) : ( Irganox 1010, made by Ciba-Geigy Japan, Ltd. ) | 0.4 parts by weight |

These components were well mixed in the melting condition at 140 °C and coated in a thickness of 150 µm on a polyethylene film of thickness 40 µm, whereby a target part was made.

### Example 2

The procedure of example 1 was repeated except that the hot melt resin composition was changed in the combination as described below.
The hot melt resin composition was,

| | |
|---|---|
| base polymer : SIS ( SL - 102, made by Nippon Zeon Co., Ltd. ) | 22 parts by weight |
| tackifier resin : hydrogenated petroleum resin ( Escorez 5300, made by Tonex Co., Ltd. ) | 25 parts by weight |
| oil : paraffin-based oil ( PW - 90, made by Idemitsu Kosan Co., Ltd. ) | 10 parts by weight |
| wax : ( Sasol H 1, made by Sasol Chemical Industries, Ltd. ) | 43 parts by weight |
| antioxidant ( age resister ) : ( Irganox 1010, made by Ciba-Geigy Japan, Ltd. ) | 0.4 parts by weight |
| ultraviolet rays absorbent ( JF - 77, made by Johoku Chemical Co., Ltd. ) | 0.2 parts by weight |

### Example 3

The procedure of example 1 was repeated except that the hot melt resin composition was changed in the combination as described below.

| | |
|---|---|
| base polymer : SBS ( Tufprene 315, made by Asahi Chemical Industry Co., Ltd. ) | 25 parts by weight |
| tackifier resin : hydrogenated petroleum resin ( Admarv S - 100, made by Idemitsu Petrochemical Co., Ltd. ) | 25 parts by weight |
| oil : paraffin-based oil ( PW - 90, made by Idemitsu Kosan Co., Ltd. ) | 5 parts by weight |
| wax : ( Sasol H 1, made by Sasol Chemical Industries, Ltd. ) | 45 parts by weight |
| antioxidant ( age resister ) : ( Irganox 1010, made by Ciba-Geigy Japan, Ltd. ) | 0.4 parts by weight |
| ultraviolet rays absorbent ( JF - 77, made by Johoku Chemical Co., Ltd. ) | 0.2 parts by weight |

### Example 4

The procedure of example 1 was repeated except that the hot melt resin composition was coated in a pattern shown in Fig. 11 ( refer to Fig. 14 ) under the conditions of a coating temperature ( a screen temperature ) of 110 °C , a coating amount of 50 g/m², and a web speed of 100 m/minute, and using a screen printing machine ( Micro Print, made by LTI GRACO Co., Ltd. ).

Besides, as carried out in the example 4, if the hot melt resin composition is coated so as to draw a mark indicating a sticking position for a tab of the target part by the resin composition, it is unnecessary to draw the mark on the base material, so that the present working process is convenient for use.

For the disposable diapers in the above examples 1 ∼ 4, the undermentioned properties of from ( 1 ) to ( 5 ) were investigated. Results are shown in Table 1.

### ( 1 ) Surface energy

It was investigated by a method of measuring a contact angle of a solid.

### ( 2 ) Adhering property with tab ( or fastening tape )

The peel strength between the tab ( for commercial articles, their respective fastening tape and, for the examples, a commercially-available adhesive tape, made by 3M Co., Ltd. ) and the target part was investigated.

### ( 3 ) Refastening property

Similarly to the ( 2 ), the sticking together and peeling-off were repeatedly carried out and the fifth repeat was used for measurement of the peel strength.

### ( 4 ) Open time

In a pilot line which shows the whole of Fig. 3, a hot melt resin composition was melted at 140°C and coated on a polyethylene film having a thickness 40 µm in a coating thickness of 150 µm, on which a paper of fine quality was stuck. The coating was carried out with varying a line speed, then, a period of time of from the coating to the sticking of the paper of fine quality was varied in a range of from 0.1 to 1.0 second, and the longest time necessary for adhesion was assigned as the open time.

### ( 5 ) Blocking property

A sample was prepared by coating the hot melt resin composition of the example on a polyethylene film and, on a resin composition side of the sample, a polyethylene film was piled up and the sample was stood for 24 hours in an oven at 60°C, and whether or not the piled polyethylene film and the resin composition stuck together was examined ( the sticking is shown by cross X and not sticking by a circle ○ ).

For comparison with the examples of the present invention, the target tape parts of the under-described commercially-available disposable papers were taken by cutting and subjected to examination of the above-described ( 1 ) ∼ ( 3 ) properties. Results are shown in Table 1.
- Comparative example 1: Pampers of Procter & Gamble Far East Inc.
- Comparative example 2: Ultra Mooney of Uni-Charm Corporation.
- Comparative example 3: Merries of Kao Corporation.

**Table 1**

| | Example 1 | Example 2 | Example 3 | Example 4 | Comparative example 1 | Comparative example 2 | Comparative example 3 |
|---|---|---|---|---|---|---|---|
| Surface energy (unit: dyne/cm²) | 19.5 | 19.1 | 22.7 | 19.7 | 20.3 | 23.5 | 20.5 |
| (unit: N/cm² (x 10⁵)) | | | | | | | |
| Adhering property with tab (unit: gf/25mm) | 460 | 430 | 450 | 480 | 420 | 440 | 400 |
| (unit: N/25mm) | 4.51 | 4.21 | 4.41 | 4.70 | 4.12 | 4.31 | 3.92 |
| Refastening property (unit: gf/25mm) | 400 | 410 | 420 | 440 | 470 | 420 | 400 |
| (unit: N/25mm) | 3.92 | 4.02 | 4.12 | 4.31 | 4.61 | 4.12 | 3.92 |
| Open time (unit: second) | 0.2 or less | 0.2 or less | 0.2 or less | 0.2 or less | ― | ― | ― |
| Blocking property | ○ | ○ | ○ | ○ | ― | ― | ― |

As seen in these results, the present examples are practically in a level of no problem and the target parts were obtained with materials in amounts less than those for commercially-available articles and also, by simpler processes.

## Claims

1. An article having a target part and a tab to attach to the target part to form an impermanent adhesion therewith which can be terminated and reestablished repeatedly, the target part having thereon a hot melt resin composition, characterized in that the hot melt resin composition has an open time of 0.5 seconds or less and comprises:
i) 20-40% by weight of a base polymer;
ii) 20-40% by weight of a tackifier resin;
iii) 30-50% by weight of Sasol Wax H1;
iv) 0-20% by weight of an oil;
and in that the coating of the hot melt resin composition on the target part has a pattern which defines alternative attachment areas on the target part for attachment of the tab.

2. An article according to Claim 1, characterised in that the article is a diaper.

3. An article as claimed in Claims 1 or Claim 2, characterised in that the target part comprises areas coated with hot melt composition interspersed with areas which are uncoated.

4. A method for producing an article as defined in any one of Claims 1 to 3, characterised in that the method comprises continuously moving the target part and applying the hot melt resin composition directly onto the continuously moving target part.

5. A method according to Claim 4, characterised in that screen coating is used to coat the hot melt resin composition onto the target part.

6. A method according to either of Claims 4 and 5, characterised in that the pattern of the hot melt resin composition is applied to the target part in a pattern which identifies the alternative attachment areas for the tab.

## Patentansprüche

1. Artikel mit einem Empfangsteil und einem Streifen zum Anbringen an das Empfangsteil, um mit diesem einen unbeständigen Klebeverschluß zu bilden, der wiederholt aufgelöst und wieder befestigt werden kann, wobei auf dem Empfangsteil eine heiße Schmelzharzzusammensetzung aufgetragen ist, dadurch gekennzeichnet, daß die heiße Schmelzharzzusammensetzung eine Öffnungszeit von 0,5 Sekunden oder weniger hat und aus folgenden Stoffen besteht:
i) 20-40 Gew.-% eines Basispolymers;
ii) 20-40 Gew.-% eines klebrigmachenden Harzes;
iii) 30-50 Gew.-% Sasol Wax H1;
iv) 0-20 Gew.-% eines Öls;
und dadurch, daß die Beschichtung der heißen Schmelzharzzusammensetzung auf dem Empfangsteil ein Muster aufweist, welches alternative Befestigungsbereiche auf dem Empfangssteil für das Befestigen des Streifens abgrenzt.

2. Artikel nach Anspruch 1, dadurch gekennzeichnet, daß der Artikel eine Windel ist.

3. Artikel nach Anspruch 1 oder Anspruch 2, dadurch gekennzeichnet, daß das Empfangsteil aus einer Mischung aus Bereichen, die mit der heißen Schmelzzusammensetzung beschichtet sind, und Bereichen, die unbeschichtet sind, besteht.

4. Verfahren zur Herstellung eines Artikels nach einem der Ansprüche 1 bis 3, dadurch gekennzeichnet, daß das Verfahren daraus besteht, das Empfangsteil kontinuierlich zu bewegen und die heiße Schmelzharzzusammensetzung direkt auf das sich kontinuierlich bewegende Empfangsteil aufzutragen.

5. Verfahren nach Anspruch 4, dadurch gekennzeichnet, daß Siebbeschichtung verwendet wird, um die heiße Schmelzharzzusammensetzung auf das Empfangsteil aufzuschichten.

6. Verfahren nach einem der Ansprüche 4 und 5, dadurch gekennzeichnet, daß das Muster der heißen Schmelzharzzusammensetzung in einem Muster auf das Empfangsteil aufgetragen wird, welches die alternativen Befestigungsbereiche für den Streifen identifiziert.

## Revendications

1. Un article ayant une partie réceptrice et une patte à fixer sur la partie réceptrice afin de former avec cette dernière une adhésion transitoire qui peut être terminée et rétablie à maintes reprises, la partie réceptrice comportant une composition de résine thermofusible, caractérisé en ce que la composition de résine thermofusible possède un temps ouvert inférieur ou égal à 0.5 secondes et comprend :
i) 20-40% en poids d'un polymère de base ;
ii) 20-40% en poids d'une résine collante ;
iii) 30-50% en poids de Cire Sasol H1 ;
iv) 0-20% en poids d'une huile ;
et en ce que l'enduction de la composition de résine thermofusible sur la partie réceptrice a un motif qui définit les zones de fixation alternatives sur la partie réceptrice pour la fixation de la patte.

2. Un article selon la Revendication 1, caractérisé en ce que l'article est une couche.

3. Un article selon la Revendication 1 ou 2, caractérisé en ce que la partie réceptrice comprend des zones enduites de composition thermofusible parsemées de zones qui ne sont pas enduites.

4. Un procédé pour fabriquer un article comme défini dans l'une des Revendications 1 à 3, caractérisé en ce que le procédé consiste à faire bouger continuellement la partie réceptrice et appliquer la composition de résine thermofusible directement sur la partie réceptrice qui bouge continuellement.

5. Un procédé selon la Revendication 4, caractérisé en ce que l'enduction par écran est utilisée pour enduire la composition de résine thermofusible sur la partie réceptrice.

6. Un procédé selon les Revendications 4 et 5, caractérisé en ce que le motif de la composition de résine thermofusible est appliqué sur la partie réceptrice selon un motif qui définie les zones de fixation alternatives pour la patte.
